# EUROPEAN PATENT APPLICATION

(11) **EP 0 581 967 A1**
(43) Date of publication of application: **09.02.1994**
(21) Application number: 93904327.9
(22) Date of filing: 22.02.1993
(51) Int. Cl.: C12N 15/12, C07K 7/10, A61K 39/02

(54) **PROTEIN WITH NOVEL PHARMACOLOGICAL CHARACTERISTIC**

(30) Priority: 21.02.1992 JP 35445/92
(71) Applicant: MOCHIDA PHARMACEUTICAL CO., LTD., Shinjuku-ku Tokyo 160 (JP)
(72) Inventor: SEYA, Tsukasa, 2-chome, Nara-shi, Nara 631 (JP); KANAMORI, Toshinori, Shinjuku-ku, Tokyo 160 (JP); NII, Atsushi, Shinjuku-ku, Tokyo 160 (JP)
(74) Representative: Grünecker, Kinkeldey, Stockmair & Schwanhäusser Anwaltssozietät
(86) International application number: JP9300207
(87) International publication number: WO9317122

(57) **Abstract**

A protein with a novel pharmacological characteristic, comprising a natural membrane cofactor protein (MCP) devoid of at least a mebrane penetration region and an intracytoplasmic region. It has not only a cofactor activity against C3b present in a liquid phase but also a cofactor activity of a I factor against C3b present in a solid phase, and is efficacious in treating diseases wherein a complement system adversely affects the cells of its own. The invention also relates to a DNA having a base sequence coding for the protein and a pharmaceutical preparation containing the protein as at least one of the active ingredients.

## Description

### TECHNICAL FIELD

This invention relates to a physiologically active protein which contains as its part the same amino acid sequence structure of a natural type membrane cofactor protein (to be referred to as MCP hereinafter) and is useful for the treatment of certain diseases in which activated complement exerts injurious actions on the host cells and tissues. More particularly, it relates to a protein having a novel pharmacological property as a cofactor necessary for factor I to inactivate C3b which is an activated fragment of the third component of complement that takes an important role in the activation of complement system. This invention also relates to a DNA fragment containing a nucleotide sequence which encodes the protein having a novel pharmacological property and to a pharmaceutical composition for use in the treatment of the aforementioned diseases, which contains the protein as an active ingredient.

### BACKGROUND OF THE INVENTION

Complement system is a bioregulation system composed of about 20 protein components contained in blood, which has been found in blood plasma in the close of the 1880's firstly as a lytic substance which is inactivated when heated at 55°C. Thereafter, a number of protein components which constitute the complement system have been isolated and identified through a series of studies which revealed that the complement system forms a cascade reaction having two different activation starting points (classical pathway and alternative pathway). The classical pathway is activated mainly by antigen-antibody complexes, while the alternative complement pathway is activated mainly by foreign substances (microorganisms and the like) which invaded the living body. Though respective complement components in both pathways undergo sequential reactions along with the cascade finally effecting lysis of target cells, both pathways differ from each other in terms of the formation mechanism of the third component of complement (to be referred to as C3 hereinafter) convertase complex and the fifth component of complement (to be referred to as C5 hereinafter) convertase complex, both being important cascade products in the system. That is, C3 convertase complex in the classical pathway is C4b2a bound to cell membrane and the like as a reaction complex of the fourth component of complement (to be referred to as C4 hereinafter) with the second component of complement (to be referred to as C2 hereinafter), while C5 convertase complex is C4b2a3b which is a reaction complex of C3 convertase complex with C3.

On the other hand, C3 convertase complex in the alternative pathway is C3bBb bound to cell membrane and the like as a reaction complex of C3 with factor B, while C5 convertase complex is C3bBb3b which is a reaction complex of the C3 convertase complex C3bBb with C3.

In both pathways, C5 (C5b) activated by the C5 convertase complex undergoes sequential reaction from C5 to C9, finally forming a membrane attack complex (to be referred to as MAC hereinafter) which causes cytolysis. In addition to these components, several other cascade products have been found in the complement system, which are possessed of various other physiological activities than the direct cytotoxic function. Especially, C3a as one of the activated products of C3 and C5a as one of the activated products of C5 are known in other name as anaphylatoxin which releases histamine from mast cells and shows chemotaxis for leucocytes. These products are considered to have a function to accelerate permeability of blood vessels thereby attracting leucocytes to an area where the complement system is being activated, which seems to be rational for the treatment of immune complexes and fragments of lysed cells by phagocytosis and the like.

While cytolytic mechanism of the complement system on bacterial cells and heterologous cells has been revealed, a question has been posed why the complement system does not exert injurious actions upon the host cells. As a clue to the question, a group of molecules have recently been found on cell membranes, which inhibit the function of autologous complement system. Especially, discovery of three cell membrane-bound proteins which bind to C3b that takes an important role in the complement cascade reaction was an epoch-making event in understanding effects of the complement system on the host cells. That is, a complement receptor 1 (to be referred to as CR1 hereinafter), a decay-accelerating factor (to be referred to as DAF hereinafter) and a membrane cofactor protein (to be referred to as MCP hereinafter) have been discovered in succession, their genes have been cloned and a number of studies have been made on their reaction characteristics and complement regulation mechanism.

CR1 was identified in 1980 as a cell membrane-bound protein of about 200 kd in its molecular weight, which has functions of both 1) acting as a cofactor of factor I in plasma to convert C3b and C4b into their inactive forms iC3b and iC4b (irreversible protein hydrolyzing reaction) and 2) strongly accelerating dissociation of C3 convertase complex (reversible complex dissociation reaction) (Fearon, D.T., *J. Exp. Med.,* 152, 20 - 30, 1980). Its gene was cloned in 1987, and, according to the analysis of the nucleotide sequence, CR1 is a transmembrane type glycoprotein composed of a total of 4 units, each unit consisting of 7 repeating structures (short consensus repeat, to be referred to as SCR hereinafter) of about 60 amino acids (Klickstein, L.B. et al., *J. Exp. Med.,* 165, 1095 - 1112, 1987).

DAF is a glycoprotein of about 70 kd in its molecular weight which has been isolated from human erythrocytes as a substance having a strong function to accelerate dissociation of C3 and C5 convertase complexes (reversible complex dissociation reaction) (Nicholson-Weller, A. et al., *J. Immunol.,* 129, 184 - 189, 1982). Its gene was cloned also in 1987, and the analysis of the gene revealed that DAF also is composed of 4 repeating structures, each of which having about 60 amino acids (Caras, I.W. et al., *Nature,* 325, 545 - 549, 1987, and Medof, M.E. et al., *Proc. Natl. Acad. Sci. USA,* 84, 2007 - 2011, 1987).

MCP is a membrane-bound glycoprotein having a molecular weight of 45 to 70 kd which has been cloned most recently among these three proteins (Lublin, D.M. et al., *J. Exp. Med.,* 168, 181 - 194, 1988). Structure of MCP resembles the DAF structure because it also is composed of 4 repeating structures each of which having about 60 amino acids, but MCP shows only an activity as a cofactor of factor I (irreversible C3b and C4b inactivation reaction) with no decay-accelerating function (Seya, T. et al., *J. Exp. Med.,* 163, 837 - 855, 1986). Human MCP is divided into at least 6 isoforms based on the difference in their alternative splicing modes of mRNA (International Publication No. WO91/02002 and Post, T.W. et al., *J. Exp. Med.,* 174, 93 - 102, 1991).

Further interestingly, it has been revealed that MCP acts on C3b which is present on MCP-expressing cells but does not act on C3b on other cells and that natural type MCP extracted and isolated from cell membranes acts on C3b contained in a liquid phase or C3b bound to a solubilized substrate but hardly acts on C3b bound to an insoluble substrate (Seya, T., *Nihon Rinshoh*, 46 (9) supplement, 1933 - 1937, 1988; Seya, T. et al., *Biochem. J.,* 264, 581 - 588, 1989; and International Publication No. WO91/02002).

As the true nature of the complement regulating proteins expressed on cell membrane has been revealed as described above, attempts have been made to apply them to agents for use in the treatment of diseases. It has been found that total complement activity in blood decreases and complement components deposit on inflammation tissues caused by the activation of the complement in various diseases (typical examples include nephritis, arteritis, SLE and the like). In consequence, since it is probable that the complement system exerts injurious actions upon the host cells under such morbid conditions, it is considered that these complement regulating proteins are applicable to effective agents for the treatment of such diseases.

With regard to CR1, soluble CR1 produced by genetic engineering techniques has been examined on its efficacies in a post-ischemic myocardial necrosis model (Weisman, H.F. et al., *Science,* 249, 146 - 151, 1990), in a reversed passive Arthus reaction model (Yeh, C.G. et al., *J. Immunol.,* 146, 250 - 256, 1991) and in an ultra-acute graft rejection model (Pruitt, S.K. et al., *J. Surg. Res.,* 50, 350 - 355, 1991), each report describing that the soluble CR1 showed an inhibitory action against each model lesion.

With regard to DAF, its therapeutic effects have been examined (Medof, M.E. et al., *Proc. Natl. Acad. Sci. U.S.A.,* 82, 2980 - 2984, 1985) using erythrocytes collected from a patient of paroxysmal nocturnal hemoglobinuria (it is known that the erythrocyte membrane of the patient is deficient in DAF, thus causing hemolysis by the autologous complement system). Such examination in the report, however, was not able to achieve complete recovery of the morbid state.

Thus, these complement regulatory proteins have recently been considered as promising substances to be applied to pharmaceutical drugs, but with several important problems still to be dissolved prior to their practical provision for use in the medical field. That is, though CR1 seems to be most promising as a therapeutic agent because its efficacies have been confirmed in various morbid models, its production and handling in purification steps cannot generally be made easily because of its enormous molecular weight of about 200 kd. As described in the foregoing, effect of DAF as a therapeutic agent is not as good as expected.

With regard to MCP, there are no reports to date on its illustrative efficacy, but it has been revealed that MCP does not act on C3b adsorbed to a solid phase substrate (insoluble substrate) (Seya, T., *Nihon Rinshoh,* 46 (9) supplement, 1933 - 1937, 1988; Seya, T. et al., *Biochem. J.,* 264, 581 - 588, 1989; and International Publication No. WO91/02002). In other words, efficacy of MCP cannot be expected so much from the prior art techniques when taken into consideration both facts that the complement exerts its cytotoxic function on the cell membrane and that, as a reaction property of natural type MCP when used extrinsically, it does not act on C3b which is adsorbed to a solid phase substrate or onto other cells but acts only in a liquid phase as a cofactor of factor I.

### DISCLOSURE OF THE INVENTION

With the aim of providing the medical field with a pharmaceutical composition for use in the treatment of certain diseases in which the complement system exerts injurious reactions upon the host cells and tissues, the present invention contemplates creating a protein having a novel pharmacological property which has a relatively small molecular weight so that its production and handling in its purification steps can be made easily and which can inhibit the action of complement components adsorbed to the actual area, namely a solid phase substrate (cell membrane), where the complement exerts its injurious function.

Accordingly, based on an assumption that the currently known natural type MCP molecule would contain a region which, when use extrinsically, inhibits the action of the molecule on C3b adsorbed to a solid phase, the inventors of the present invention have considered that deletion of the inhibitory region could result in the addition of an important pharmacological property to the molecule, namely an ability to react with C3b on the solid phase. On the basis of such a new idea, the inventors of the present invention have conducted intensive studies and succeeded in creating a protein having a novel pharmacological property, thereby accomplishing the present invention.

According to a first aspect of the present invention, there is provided a protein having a novel pharmacological property which comprises a natural type membrane cofactor protein (MCP) from which at least its transmembrane region and cytoplasmic tail region are deleted.

Preferably, the protein of the first aspect of the present invention may contain at least a portion of an amino acid sequence represented by formula 1 which will be described later.

According to a second aspect of the present invention, there is provided a DNA fragment containing a nucleotide sequence which encodes the protein of the first aspect of the present invention having a novel pharmacologiocal property.

According to a third aspect of the present invention, there is provided a pharmaceutical composition for use in the treatment of certain diseases in which the complement system exerts injurious reaction upon the host cells, which contains the protein of the first aspect of the present invention having a novel pharmacologiocal property as at least one of its active ingredients.

### BRIEF DESCRIPTION OF THE DRAWINGS

Fig. 1 is an illustration showing 6 isoform structures of human MCP.

Fig. 2 is a schematic illustration showing construction steps of pUC19-sMCP (C).

Fig. 3 is a schematic illustration showing construction steps of pUC19-sMCP (D).

Fig. 4 is a schematic illustration showing construction steps of pUC19-sMCP1234.

Fig. 5 is a schematic illustration showing construction steps of a expression plasmid for COSI .

Fig. 6 is a graph showing SDS-PAGE band patterns which indicate cofactor activities.

Fig. 7 is a graph showing the number of cells which indicate effect on the complement-dependent cytotoxicity.

### CONSTRUCTION OF THE INVENTION

The following describes the present invention in detail.

The protein of the first aspect of the present invention is a protein having a novel pharmacological property which comprises a natural type membrane cofactor protein (MCP) from which at least its transmembrane region and cytoplasmic tail region are deleted.

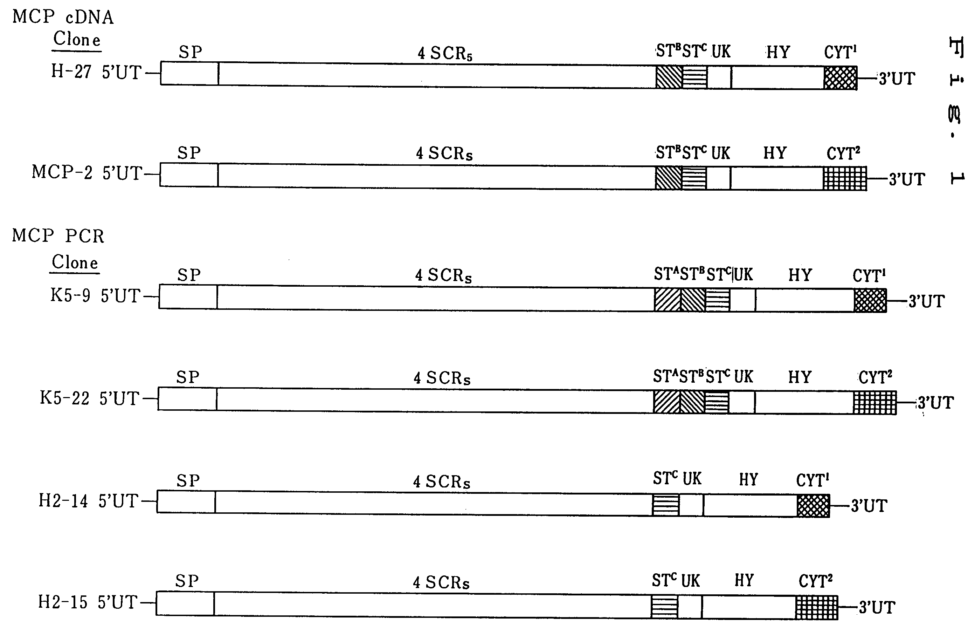

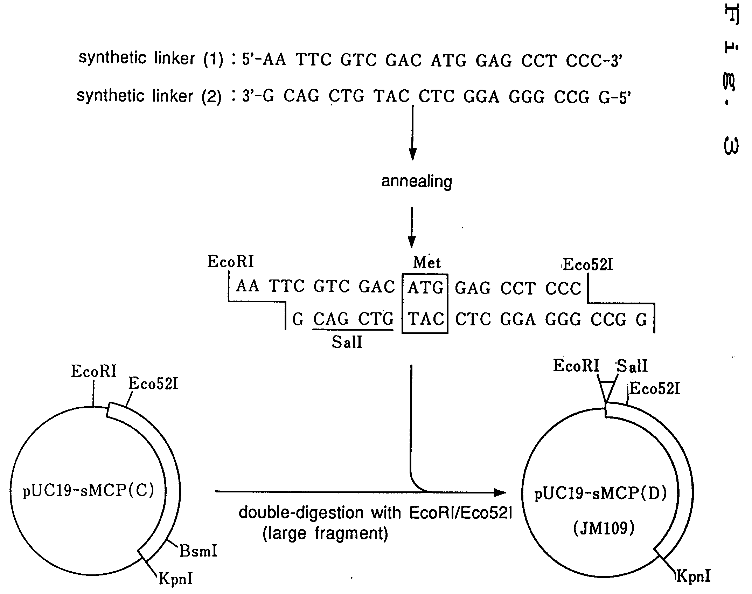

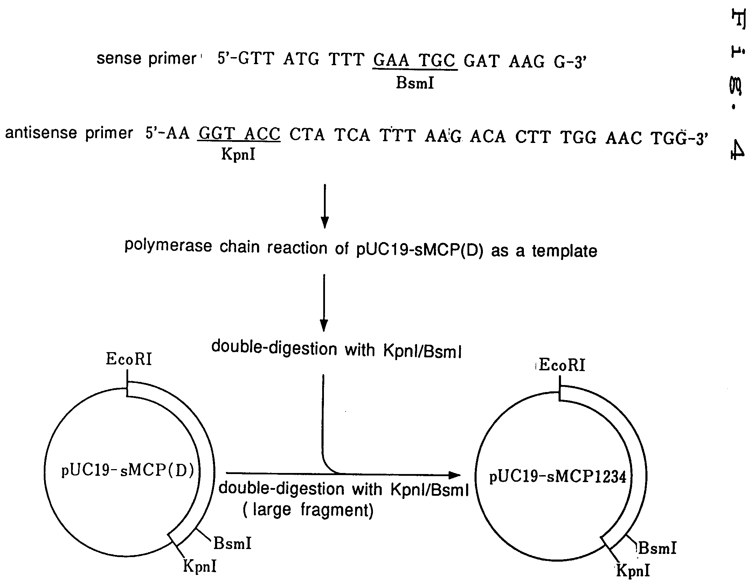

Fig. 1 is an illustration quoted from Fig. 3 disclosed in International Publication No. WO91/02002, which shows 6 isoform structures of MCP given by cDNA clones. In Fig. 1, 4SCRs indicates 4 SCR regions, ST (ST^{A}, ST^{B} and ST^{C}) indicates a serine-threonine rich region, UK indicates a UK (unknown) region whose sequence function is unknown, HY is a hydrophobic region and CYT (CYT¹ and CYT²) is a cytoplasmic tail region).

The aforementioned transmembrane region of natural type MCP means a membrane-penetrating portion of the natural type MCP which exists generally on a cell membrane partly penetrating into the membrane, and this region corresponds to the area mentioned as HY in Fig. 1. Also, the cytoplasmic tail region corresponds to the area mentioned as CYT¹ or CYT² in Fig. 1. With reference to the amino acid sequence shown in Fig. 1 which is disclosed in International Publication No. WO91/02002, amino acid numbers 295 - 317 correspond to the transmembrane region and numbers 318 - 350 correspond to the cytoplasmic tail region.

The protein of the first aspect of the present invention from which at least its transmembrane region and cytoplasmic tail region are deleted corresponds to a protein coded by the MCP clone shown in Fig. 1 from which a C terminal side amino acid sequence starting from the protein regions corresponding to ST (ST^{A}, ST^{B}, ST^{C}) and UK, or from an optional point in both end areas of these regions, is deleted. Illustrative examples include a protein having a [4SCRs-ST^{C}-UK] structure, a protein having a [4SCRs-ST^{A}-ST^{B}-ST^{C}] structure, a protein having a [4SCRs-ST^{C}] structure, a protein having a [4SCRs] structure and the like.

As shown in Fig. 1, it has been revealed that at least 6 isoforms exist in MCP based on the difference in the alternative splicing of mRNA (International Publication No. WO91/02002 and Post, T.W. et al., *J. Exp. Med.,* 174, 93 - 102, 1991). Among the proteins of the present invention having novel activity which have been created based on a new idea, the one shown in the following formula 1 (Sequence ID No. 1) was obtained by deleting amino acid numbers -34 - -1, 252 - 266 and 295 and subsequent numbers from the amino acid sequence shown in Fig. 1 disclosed in the specification of International Publication No. WO91/02002, and the one shown in the following formula 2 (Sequence ID No. 2) was obtained by deleting amino acid numbers -34 - -1 and 252 and subsequent numbers from the amino acid sequence shown in Fig. 1 disclosed in the specification of International Publication No. WO91/02002.

Particularly, the protein of the present invention may contain at least a portion of the amino acid sequence shown in the following formula 1, may contain the amino acid sequence of the following formula 1 or 2, or may be the amino acid sequence itself of the following formula 1 or 2. 
(Formula 1)

Cys Glu Glu Pro Pro Thr Phe Glu Ala Met Glu Leu Ile Gly Lys Pro
1               5                   10                  15

Lys Pro Tyr Tyr Glu Ile Gly Glu Arg Val Asp Tyr Lys Cys Lys Lys
            20                  25                  30

Gly Tyr Phe Tyr Ile Pro Pro Leu Ala Thr His Thr Ile Cys Asp Arg
        35                  40                  45

Asn His Thr Trp Leu Pro Val Ser Asp Asp Ala Cys Tyr Arg Glu Thr
    50                  55                  60

Cys Pro Tyr Ile Arg Asp Pro Leu Asn Gly Gln Ala Val Pro Ala Asn
65                  70                  75                  80

Gly Thr Tyr Glu Phe Gly Tyr Gln Met His Phe Ile Cys Asn Glu Gly
                85                  90                  95

Tyr Tyr Leu Ile Gly Glu Glu Ile Leu Tyr Cys Glu Leu Lys Gly Ser
            100                 105                 110

Val Ala Ile Trp Ser Gly Lys Pro Pro Ile Cys Glu Lys Val Leu Cys
        115                 120                 125

Thr Pro Pro Pro Lys Ile Lys Asn Gly Lys His Thr Phe Ser Glu Val
    130                 135                 140

Glu Val Phe Glu Tyr Leu Asp Ala Val Thr Tyr Ser Cys Asp Pro Ala
 
145                 150                 155                 160

Pro Gly Pro Asp Pro Phe Ser Leu Ile Gly Glu Ser Thr Ile Tyr Cys
                165                 170                 175

Gly Asp Asn Ser Val Trp Ser Arg Ala Ala Pro Glu Cys Lys Val Val
            180                 185                 190

Lys Cys Arg Phe Pro Val Val Glu Asn Gly Lys Gln Ile Ser Gly Phe
        195                 200                 205

Gly Lys Lys Phe Tyr Tyr Lys Ala Thr Val Met Phe Glu Cys Asp Lys
    210                 215                 220

Gly Phe Tyr Leu Asp Gly Ser Asp Thr Ile Val Cys Asp Ser Asn Ser
225                 230                 235                 240

Thr Trp Asp Pro Pro Val Pro Lys Cys Leu Lys Gly Pro Arg Pro Thr
                245                 250                 255

Tyr Lys Pro Pro Val Ser Asn Tyr Pro Gly Tyr Pro Lys Pro Glu Glu
            260                 265                 270

Gly Ile Leu Asp Ser Leu Asp
        275                 279

(Formula 2)

Cys Glu Glu Pro Pro Thr Phe Glu Ala Met Glu Leu Ile Gly Lys Pro
  1               5                  10                  15

Lys Pro Tyr Tyr Glu Ile Gly Glu Arg Val Asp Tyr Lys Cys Lys Lys
             20                  25                  30

Gly Tyr Phe Tyr Ile Pro Pro Leu Ala Thr His Thr Ile Cys Asp Arg
         35                  40                  45

Asn His Thr Trp Leu Pro Val Ser Asp Asp Ala Cys Tyr Arg Glu Thr
     50                  55                  60

Cys Pro Tyr Ile Arg Asp Pro Leu Asn Gly Gln Ala Val Pro Ala Asn
 65                  70                  75                  80
 
Gly Thr Tyr Glu Phe Gly Tyr Gln Met His Phe Ile Cys Asn Glu Gly
                85                  90                  95

Tyr Tyr Leu Ile Gly Glu Glu Ile Leu Tyr Cys Glu Leu Lys Gly Ser
            100                 105                 110

Val Ala Ile Trp Ser Gly Lys Pro Pro Ile Cys Glu Lys Val Leu Cys
        115                 120                 125

Thr Pro Pro Pro Lys Ile Lys Asn Gly Lys His Thr Phe Ser Glu Val
    130                 135                 140

Glu Val Phe Glu Tyr Leu Asp Ala Val Thr Tyr Ser Cys Asp Pro Ala
145                 150                 155                 160

Pro Gly Pro Asp Pro Phe Ser Leu Ile Gly Glu Ser Thr Ile Tyr Cys
                165                 170                 175

Gly Asp Asn Ser Val Trp Ser Arg Ala Ala Pro Glu Cys Lys Val Val
            180                 185                 190

Lys Cys Arg Phe Pro Val Val Glu Asn Gly Lys Gln Ile Ser Gly Phe
        195                 200                 205

Gly Lys Lys Phe Tyr Tyr Lys Ala Thr Val Met Phe Glu Cys Asp Lys
    210                 215                 220

Gly Phe Tyr Leu Asp Gly Ser Asp Thr Ile Val Cys Asp Ser Asn Ser
225                 230                 235                 240

Thr Trp Asp Pro Pro Val Pro Lys Cys Leu Lys
                245                 250 251
Different greatly from the generally known MCP, the protein having a novel pharmacological property according to the first aspect of the present invention may preferably be a protein having a novel pharmacological property, characterized in that, similar to the case of its reaction in a liquid phase, it can react as a cofactor of factor I with a solid phase C3b, with which the prior art counterpart protein cannot react. The reaction as a cofactor of factor I means an active reaction in an inactivation reaction of C3b in the complement system, namely in a conversion reaction of C3b into iC3b, more illustratively, it means that extrinsically supplied protein of the present invention acts as a cofactor of factor I which is one of the complement system regulatory factors and, as the results, irreversibly hydrolyzes the complement component C3b thereby inhibiting or extinguishing the function of C3b.

This phenomenon overthrows the common knowledge in the prior art field, because it was not thinkable by any person skilled in the art. In fact, in the specification of International Publication No. WO91/02002, its inventors disclose a soluble type MCP, but with no recognition or assumption about such a novel pharmacological property, and conversely disclose that the soluble type MCP has no function as a cofactor of factor I against C3b on a solid phase. In consequence, not only the MCP disclosed in the specification of the present invention is different from the one disclosed in International Publication No. WO91/02002, but also there is a line drawn between them.

The term C3b on a solid phase means a state in which the activated complement component C3b binds, via a covalent or non-covalent bonding, to biological tissues and cells or a living body-constituting tangible component or an artificial tangible material artificially arranged in the living body, with an example being C3b which exists on a solid phase such as cell membrane or on an insoluble substrate in an adsorbed or bound state. Examples of the C3b in a liquid phase include C3b which exists in the body fluid or in various types of solutions in the free state and C3b bound to a solubilized substrate.

The protein of the present invention having a novel pharmacological property has been created on the basis of a new idea of removing a portion of the known MCP, a portion which hinders the action of MCP upon C3b on a solid phase, and the inventive protein may therefore be in any form provided that it can show an activity against C3b on a solid phase similar to the case of the cofactor activity against C3b in a liquid phase. A mutation such as deletion, addition or substitution can be introduced into the amino acid sequence of the protein of the present invention without altering its basic properties (physical properties, activities and the like), especially its function against C3b on a solid phase. Examples of the presumable mutation include substitution of a hydrophobic amino acid residue with other hydrophobic amino acid residue, substitution of an amino acid residue having positive charge with other amino acid residue having positive charge, mutual substitution of Glu and Asp, or Lys, His and Arg, and substitution among groups of Ile, Val, Met and Leu, groups of Gly, Ala, Ser and Cys and groups of Trp, Tyr and Phe. In consequence, mutants of a protein defined by the amino acid sequence itself of the aforementioned formula 1 or 2 or a protein having at least the amino acid sequence of the aforementioned formula 1 or 2, resulting from substitution, deletion, addition or the like of at least one amino acid in N-terminus and/or C-terminus and/or inner part of the protein, are included in the protein of the present invention provided that the aforementioned basic properties of the protein of the present invention having a novel pharmacological property can be recognized. In addition, as a matter of course, chemically modified products of such mutants by various means such as glycosylation, alkylation, oxidation, reduction, hydrolysis and the like and those formed into salts with pharmacologically acceptable acids or bases are also included in the protein of the present invention having a novel pharmacological property.

The protein having a novel pharmacological property according to the first aspect of the present invention may be obtained in accordance with any typical polypeptide synthesis method such as the Merrifield's method, or from a natural MCP containing the protein molecule of the present invention, but preferably produced from cultured cells by means of genetic engineering techniques making use of a DNA fragment which will be described later as a second aspect of the present invention. More preferably, such cultured cells may be selected from *Escherichia coli* and mammalian cultured cells. It may also be obtained by means of genetic engineering techniques using at least a portion of a DNA fragment containing a nucleotide sequence of the following formula 3 (Sequence ID No. 3), namely a nucleotide sequence derived from the MCP nucleotide sequence disclosed in the specification of International Publication No. WO91/02002 by the deletion of its nucleotide sequence moiety which corresponds to the inhibitory region of the reaction with C3b on a solid phase. 
(Formula 3)

TGTGAGGAGC CACCAACATT TGAAGCTATG GAGCTCATTG GTAAACCAAA ACCCTACTAT 60

GAGATTGGTG AACGAGTAGA TTATAAGTGT AAAAAAGGAT ACTTCTATAT ACCTCCTCTT 120

GCCACCCATA CTATTTGTGA TCGGAATCAT ACATGGCTAC CTGTCTCAGA TGACGCCTGT 180

TATAGAGAAA CATGTCCATA TATACGGGAT CCTTTAAATG GCCAAGCAGT CCCTGCAAAT 240

GGGACTTACG AGTTTGGTTA TCAGATGCAC TTTATTTGTA ATGAGGGTTA TTACTTAATT 300

GGTGAAGAAA TTCTATATTG TGAACTTAAA GGATCAGTAG CAATTTGGAG CGGTAAGCCC 360

CCAATATGTG AAAAGGTTTT GTGTACACCA CCTCCAAAAA TAAAAAATGG AAAACACACC 420

TTTAGTGAAG TAGAAGTATT TGAGTATCTT GATGCAGTAA CTTATAGTTG TGATCCTGCA 480

CCTGGACCAG ATCCATTTTC ACTTATTGGA GAGAGCACGA TTTATTGTGG TGACAATTCA 540

GTGTGGAGTC GTGCTGCTCC AGAGTGTAAA GTGGTCAAAT GTCGATTTCC AGTAGTCGAA 600

AATGGAAAAC AGATATCAGG ATTTGGAAAA AAATTTTACT ACAAAGCAAC AGTTATGTTT 660

GAATGCGATA AGGGTTTTTA CCTCGATGGC AGCGACACAA TTGTCTGTGA CAGTAACAGT 720

ACTTGGGATC CCCCAGTTCC AAAGTGTCTT AAAGGTCCTA GGCCTACTTA CAAGCCTCCA 780

GTCTCAAATT ATCCAGGATA TCCTAAACCT GAGGAAGGAA TACTTGACAG TTTGGATTGA 840
When the protein of the present invention is produced by genetic engineering means, any of the usually used host-vector systems may be used. For example, when *E. coli* is used as a host, LacUV5 promoter, tryptophan promoter, λP_{L} promoter or the like may be used as a promoter. The inventive protein may be obtained as a fused protein with β-galactosidase making use of a λ phage vector, λgt11.

When a mammal culture cell line is used as a host, simian COS cells, Chinese hamster CHO cells, mouse 3T3 cells or human fibroblasts may be used preferably. When these cells are used as the host, SV40 early promoter, adenovirus major late promoter, β-actin promoter, polypeptide chain elongation factor promoter or the like may be used as a promoter. When these host-vector systems are used, the protein having a novel pharmacological property according to the present invention can be produced by culturing the host under its optimum growth conditions and giving a condition under which the promoter used can function properly.

Though not particularly limited, the protein having a novel pharmacological property according to the present invention may preferably be in the form of purified protein.

The thus obtained protein having a novel activity may be purified making use of usually used techniques for the purification of proteinous materials. For example, purification of the inventive protein may be effected by employing optional combinations of various techniques such as salting out, acid·alkali precipitation, ultrafiltration, ion exchange chromatography, gel filtration chromatography, hydrophobic chromatography, adsorption chromatography, affinity chromatography, HPLC, chromatofocusing, electrophoresis and the like. For example, a solution containing the protein of the present invention is dialyzed against a 0.02 M NaCl/10 mM phosphate buffer (pH 7.5) and applied to an ion exchange chromatography, subsequently carrying out elution of the protein by a linear gradient of NaCl. Active fractions are collected and applied to an affinity chromatography making use of a monoclonal antibody. Thereafter, the active fraction is obtained by carrying out elution with a glycine-HCl buffer (pH 2.5). Alternatively, it may be carried out in accordance with the known MCP purification method (Seya, T. et al., *J. Exp. Med.,* 163, 837 - 855, 1986). The MCP activity may be determined by confirming inactivation of C3b in the presence of factor I, namely conversion of C3b into iC3b. For example, a fluorescence-labeled C3b is prepared in accordance with the method of Seya *et al.* (Seya, T. et al., *Clin. Chim. Acta,* 119, 189 - 196, 1982), and MCP molecules are added to the thus prepared labeled substrate in the presence of factor I. After completion of the reaction, the reaction mixture is subjected to reduction with β-mercaptoethanol or the like and then to sodium dodecyl sulfate-polyacrylamide electrophoresis (to be referred to as SDS-PAGE hereinafter). Thereafter, the resulting gel is scanned on a fluorometer to confirm conversion state of C3b into iC3b based on the difference in their molecular weights.

According to a second aspect of the present invention, there is provided a DNA fragment containing a nucleotide sequence which encodes the protein of the first aspect of the present invention having a novel pharmacological property. Preferably, the DNA fragment of the second aspect of the present invention may contain at least a portion of the nucleotide sequence represented by the following formula 3 (Sequence ID No. 3), or entire length of the nucleotide sequence represented by the following formula 3 or the following formula 4 (Sequence ID No. 4), or, more preferably, it may be defined by the nucleotide sequence of the following formula 3 or 4. 
(Formula 3)

TGTGAGGAGC CACCAACATT TGAAGCTATG GAGCTCATTG GTAAACCAAA ACCCTACTAT 60

GAGATTGGTG AACGAGTAGA TTATAAGTGT AAAAAAGGAT ACTTCTATAT ACCTCCTCTT 120

GCCACCCATA CTATTTGTGA TCGGAATCAT ACATGGCTAC CTGTCTCAGA TGACGCCTGT 180

TATAGAGAAA CATGTCCATA TATACGGGAT CCTTTAAATG GCCAAGCAGT CCCTGCAAAT 240

GGGACTTACG AGTTTGGTTA TCAGATGCAC TTTATTTGTA ATGAGGGTTA TTACTTAATT 300

GGTGAAGAAA TTCTATATTG TGAACTTAAA GGATCAGTAG CAATTTGGAG CGGTAAGCCC 360

CCAATATGTG AAAAGGTTTT GTGTACACCA CCTCCAAAAA TAAAAAATGG AAAACACACC 420

TTTAGTGAAG TAGAAGTATT TGAGTATCTT GATGCAGTAA CTTATAGTTG TGATCCTGCA 480

CCTGGACCAG ATCCATTTTC ACTTATTGGA GAGAGCACGA TTTATTGTGG TGACAATTCA 540

GTGTGGAGTC GTGCTGCTCC AGAGTGTAAA GTGGTCAAAT GTCGATTTCC AGTAGTCGAA 600

AATGGAAAAC AGATATCAGG ATTTGGAAAA AAATTTTACT ACAAAGCAAC AGTTATGTTT 660

GAATGCGATA AGGGTTTTTA CCTCGATGGC AGCGACACAA TTGTCTGTGA CAGTAACAGT 720

ACTTGGGATC CCCCAGTTCC AAAGTGTCTT AAAGGTCCTA GGCCTACTTA CAAGCCTCCA 780

GTCTCAAATT ATCCAGGATA TCCTAAACCT GAGGAAGGAA TACTTGACAG TTTGGATTGA 840

(Formula 4)

TGTGAGGAGC CACCAACATT TGAAGCTATG GAGCTCATTG GTAAACCAAA ACCCTACTAT 60

GAGATTGGTG AACGAGTAGA TTATAAGTGT AAAAAAGGAT ACTTCTATAT ACCTCCTCTT 120

GCCACCCATA CTATTTGTGA TCGGAATCAT ACATGGCTAC CTGTCTCAGA TGACGCCTGT 180

TATAGAGAAA CATGTCCATA TATACGGGAT CCTTTAAATG GCCAAGCAGT CCCTGCAAAT 240

GGGACTTACG AGTTTGGTTA TCAGATGCAC TTTATTTGTA ATGAGGGTTA TTACTTAATT 300

GGTGAAGAAA TTCTATATTG TGAACTTAAA GGATCAGTAG CAATTTGGAG CGGTAAGCCC 360
 
CCAATATGTG AAAAGGTTTT GTGTACACCA CCTCCAAAAA TAAAAAATGG AAAACACACC 420
TTTAGTGAAG TAGAAGTATT TGAGTATCTT GATGCAGTAA CTTATAGTTG TGATCCTGCA 480
CCTGGACCAG ATCCATTTTC ACTTATTGGA GAGAGCACGA TTTATTGTGG TGACAATTCA 540
GTGTGGAGTC GTGCTGCTCC AGAGTGTAAA GTGGTCAAAT GTCGATTTCC AGTAGTCGAA 600
AATGGAAAAC AGATATCAGG ATTTGGAAAA AAATTTTACT ACAAAGCAAC AGTTATGTTT 660
GAATGCGATA AGGGTTTTTA CCTCGATGGC AGCGACACAA TTGTCTGTGA CAGTAACAGT 720
ACTTGGGATC CCCCAGTTCC AAAGTGTCTT AAATGA 756
The nucleotide sequence represented by the formula 3 corresponds to the one shown in Fig. 3 which is disclosed in the specification of International Publication No. WO91/02002, namely a sequence derived from the clone H2-15 shown in Fig. 1 of the present invention by the deletion of its signal peptide region (SP), hydrophobic region (HY) and cytoplasmic tail region CYT². The sequence of the formula 4 corresponds to 4 SCR regions (4SCRs) of the natural type MCP.

The DNA fragment of the present invention may further contain a nucleotide sequence which corresponds to a natural MCP signal peptide or the like.

The DNA fragment of the second aspect of the present invention may be obtained by synthesizing its complete length by usually used DNA synthesis means, or by introducing a translation termination codon into an appropriate site of the known MCP gene by means of a site-specific nucleotide sequence mutagenesis. Preferably, it may be obtained by preparing cDNA in accordance with a known method for the cloning of MCP-encoding mRNA (Lublin, D.M. et al., *J. Exp. Med.,* 168, 181 - 194, 1988), and then eliminating unnecessary portions from the cDNA and reconstructing the resulting fragments making use of genetic engineering techniques.

For example, a gene fragment which encode C-terminal side of amino acid sequence to which a translation termination codon is artificially added is prepared by a gene amplification method (polymerase chain reaction, to be referred to as PCR hereinafter) using the clone H2-14 or H2-15 shown in Fig. 1 as a template and a partial mismatch primer having a translation termination codon just before its hydrophobic region. Thereafter, the thus prepared fragment is digested with appropriate restriction enzymes and then ligated with an N-terminal side gene fragment which has been treated with restriction enzymes in the same manner.

The DNA fragment of the second aspect of the present invention may contain any nucleotide sequence provided that the DNA fragment encodes the MCP of the first aspect of the present invention and can express it under appropriate conditions, or it may be a DNA fragment which is defined by the nucleotide sequence represented by the aforementioned formula 3 or 4 or a derivative thereof in which at least one optional base is added or substituted at the 5' end and/or 3' end and/or a intermediate site of the nucleotide sequence. In the technical field of genetic engineering, it is known that a base in a nucleotide sequence of a gene can be substituted by other base based on the degeneracy of genetic code without altering an amino acid sequence of a protein encoded by the gene. That is, almost all of amino acids are encoded by a plurality of genetic codes, and, for example, Val is encoded by any of the codons GTT, GTA, GTC and GTG, and Ala by GCA, GCT, GCC and GCG. In consequence, the DNA fragment of the second aspect of the present invention also includes its mutants in which at least one base in its nucleotide sequence is substituted by other base, provided that such a substitution does not alter the encoded amino acid sequence. An *E. coli* strain, [HB101 (pM851)], transformed with a plasmid (pM851) containing a DNA fragment having the nucleotide sequence represented by the Sequence ID No. 3 and 4, has been deposited by the present inventors in Fermentation Research Institute, Agency of Industrial Science and Technology, the Ministry of International Trade and Industry [this strain has been assigned the designation as Bikokenkinki No. P-12715 which was then transferred to the International Depositary Authority as Bikokenjoki No. FERM BP-4195].

According to a third aspect of the present invention, there is provided a pharmaceutical composition for use in the treatment of certain diseases in which the complement system injuriously acts upon the host cells, which contains the protein having a novel pharmacological property of the first aspect of the present invention as at least one of its active ingredients. The pharmaceutical composition of the present invention may comprise the first aspect protein alone or contain usually used pharmaceutical additives such as fillers, stabilizers, solubilizing agents and the like, provided that they do not spoil activity of the protein upon C3b on a solid phase. Examples of such additives include Ringer solution, phosphate buffer, human serum albumin, hydrolyzed gelatin, sucrose, dextran, polyethylene glycol and the like, which may be optionally selected and used depending on the drug form.

Dose of the pharmaceutical composition of the present invention may be in the range of generally from 1 ng/kg to 10 mg/kg, preferably from 1 µg/kg to 10 mg/kg, more preferably from 10 µg/kg to 5 mg/kg, though the range of dose should be adjusted optionally depending on the age, body weight and sex of each patient and the type and degree of each disease to be treated. Such diseases to be treated are those in which the complement system acts injuriously upon the host cells and tissues, with preferred examples including nephritis, hepatitis, arteritis, asthma, autoimmune disease, ischemic organ disorder, reperfusion injury, rheumatoid arthritis, graft rejection, shock, multiorgan failure, disseminated intravascular coagulation syndrome and adult respiratory distress syndrome (ARDS), as well as ARDS-like disease and shock caused by hemodialysis and burn shock.

Treatment of diseases making use of the pharmaceutical composition of the third aspect of the present invention may be effected through any route of administration, provided that it can deliver effective dose of the protein having a novel pharmacological property of the present invention to the affected part to be treated. For example, the treatment method may be effected by a systemic administration such as intravenous injection, intraarterial administration, subcutaneous injection, intramuscular injection, oral administration or the like, by a topical application such as intraarticular injection, intraspinal injection or the like, by a transmucosal absorption such as nasal application, intratracheal injection, intraoral application or the like, or by a rectal administration in the form of suppositories or the like.

### EXAMPLES

The following examples are provided to further illustrate the present invention. It is to be understood, however, that the examples are for purpose of illustration only and are not intended as a definition of the limits of the invention. Unless otherwise noted, technical terms and abbreviations as used in the following examples are based on those which are commonly used in the related technical field. Basic procedures with regard to the recombinant DNA techniques were carried out in the light of Maniatis, T. et al., "*Molecular Cloning, A Laboratory Manual"* (Cold Spring Harbor Laboratory, 1989), and those regarding protein purification were carried out in the light of *"New Biochemical Experimental Course,* vol. 1, Isolation, Purification and Property" (edited by Japanese Biochemical Society, published by Tokyo Kagaku Dojin, 1990). Instruments and reagents were handled in accordance with each of the manufacturer's instructions.

### (Example 1) Modification of C terminal-corresponding region of MCP gene 1

In order to produce the protein having a novel pharmacological property of the present invention by means of gene engineering techniques, an MCP gene cloned in a plasmid vector pUC19 (this clone is to be called pUC19-MCP (D) hereinafter) was modified. The MCP structural gene used herein corresponds to the clone H2-15 shown in Fig. 1 and is the same gene disclosed by Post, T.W. et al. in Fig. 4 in *J. Exp. Med.,* 174, 93 - 102, 1991.

Firstly, the following two single-stranded DNA fragments were synthesized using a DNA synthesizer (Model 381A, Applied Biosystems) as primers for PCR.

### Sense primer:

5'-GTTATGTTTGAATGCGATAAGG-3'

### Antisense primer:

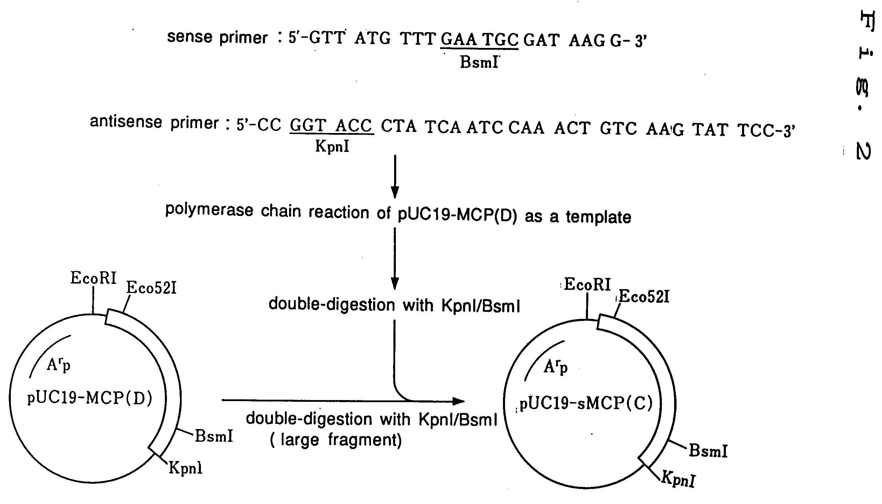

5'-CCGGTACCCTATCAATCCAAACTGTCAAGTATTCC-3'
In a 100 µl solution of 10 mM Tris-HCl (pH 8.3)/50 mM KCl/1.5 mM MgCl₂/0.01% gelatin were dissolved 0.8 µg of each of the above two primers, 50 ng of pUC19-MCP (D) as a template, 2.5 units of a thermostable DNA polymerase (Perkin-Elmer-Cetus) and 200 µM in final concentration of dNTP (N means all 4 of A, T, C and G), subsequently carrying out PCR (polymerase chain reaction) using an incubator for PCR (Thermal Cycler, Perkin-Elmer-Cetus). The reaction was carried out in accordance with the procedure of Michael, A.I. et al. *"PCR Protocols, A Guide to Methods and Applications",* Academic Press, 1990. After completion of the reaction, an amplified DNA fragment of about 190 bp was obtained by phenol/chloroform extraction and ethanol precipitation. This fragment was double-digested with restriction enzymes *Kpn*I (Takara Shuzo Co., Ltd.) and *Bsm*I (New England Biolabs, Inc.), and, using a DNA ligase (Takara Shuzo Co., Ltd.), the resulting fragment was ligated with a pUC19-MCP (D) large fragment which has been double-digested with the same restriction enzymes. The plasmid thus obtained was named pUC19-sMCP (C). The procedure of Example 1 is briefly shown in Fig. 2.

### (Example 2) Modification of an MCP gene region corresponding to an N terminal nearby area

With the aim of improving workability of recombinant DNA techniques, modification of an MCP gene region corresponding to an N terminal nearby area was carried out. Firstly, in order to obtain artificial linkers, the following two single-stranded DNA fragments were synthesized in the same manner as described in Example 1.

### Synthetic linker (1)

### upper strand:

5'-AATTCGTCGACATGGAGCCTCCC-3'

### Synthetic linker (2)

### lower strand:

3'-GCAGCTGTACCTCGGAGGGCCGG-5'
After annealing, the resulting product was ligated in the same manner as described in Example 1 with a large fragment which has been obtained by double-digesting the pUC19-sMCP (C) of Example 1 with restriction enzymes *Eco*RI (Takara Shuzo Co., Ltd.) and *Eco*52I (Takara Shuzo Co., Ltd.). The plasmid thus obtained was named pUC19-sMCP (D). The procedure of Example 2 is briefly shown in Fig. 3.

### (Example 3) Modification of C terminal-corresponding region of MCP gene 2

Using the plasmid pUC19-sMCP (D) obtained in Example 2, an MCP gene having further modified region corresponding to the C terminal area was prepared. Firstly, the following two primers were synthesized in the same manner as described in Example 1.

### Sense primer:

5'-GTTATGTTTGAATGCGATAAGG-3'

### Antisense primer:

5'-AAGGTACCCTATCATTTAAGACACTTTGGAACTGG-3'
Using these primers, and using pUC19-sMCP (D) as a template, PCR was carried out in the same manner as described in Example 1. The thus obtained fragment of about 100 bp was double-digested with restriction enzymes *Kpn*I (op. cit.) and *Bsm*I (op. cit.), and the resulting fragment was ligated with a pUC19-sMCP (D) large fragment which has been digested with the same restriction enzymes. The plasmid thus obtained was named pUC19-sMCP 1234. The procedure of Example 3 is briefly shown in Fig. 4.

### (Example 4) Construction of expression plasmid for use in COSI cells

An expression plasmid, pEFN-I, in COSI cells was constructed by the modification of pEF-BOS which is known as an efficient expression vector in mammalian cultured cells (Mizushima, S. et al., *Nucleic Acids Res.,* 18, 5322, 1990). The pEFN-I vector contains replication origin (ori) of SV40, promoter region of human polypeptide elongation factor 1α and poly A addition signal sequence of SV40.

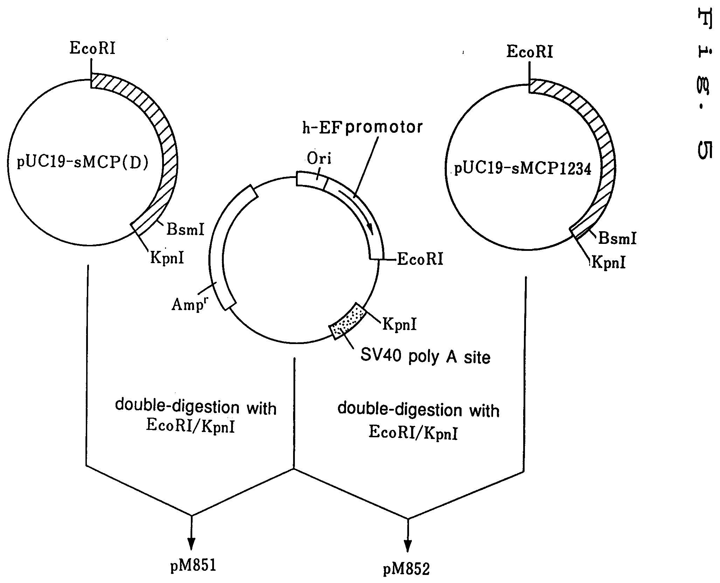

Plasmid pUC19-sMCP (D) obtained in Example 2 or pUC19-sMCP 1234 obtained in Example 3 was double-digested with restriction enzymes *Eco*RI and *Kpn*I to isolate an MCP structural gene fragment (small fragment) contained in each plasmid. Each of these fragments was ligated in the same manner as described in Example 1 with pEFN-I which has been double-digested with the same restriction enzymes. The plasmids thus obtained were named pM851 and pM852. The procedure of Example 4 is briefly shown in Fig. 5.

The plasmid pM851 contains the nucleotide sequence represented by Sequence ID No. 3, and the plasmid pM852 contains the nucleotide sequence represented by Sequence ID No. 4.

### (Example 5) Expression in COSI cells

Expression of the protein of the present invention was effected by introducing each of the plasmids pM851 and pM852 prepared in Example 4 into COSI cells. That is, a 0.5 µg portion of each of pM851 or pM852 was dissolved in 5 µl of a 10 mM Tris hydrochloride buffer (to be referred to as Tris-HCl hereinafter) (pH 7.4)/1 mM ethylenediaminetetraacetic acid (to be referred to as EDTA hereinafter) solution, subsequently adding 700 µl of Dulbecco's modified Eagle's medium (D-ME medium, Nissui Pharmaceutical Co., Ltd.) containing 0.2 mg/ml of DEAE-dextran and 50 mM of Tris-HCl (pH 7.4), thereby preparing a DNA-DEAE dextran mixture. The thus prepared DNA-DEAE dextran mixture was added dropwise to COSI cells which have been monolayer-cultured to a semi-confluent level in a culture dish of 3.5 cm in diameter, and the culturing was continued at 37°C in an atmosphere of 5% CO₂/95% air. After 4 hours culture, the DNA-DEAE dextran mixture was removed, and D-ME medium containing 1% fetal bovine serum (IRVINE SCIENTIFIC) was added to the resulting dish. After 24 hours culture, the medium was exchanged with serum-free D-ME medium, and the culturing was continued for additional 48 to 96 hours. Thereafter, the resulting culture supernatant was recovered and used in the purification of the inventive protein having a novel pharmacological property.

### (Example 6) Purification of the inventive protein from culture supernatant

The culture supernatant obtained in Example 5 was dialyzed overnight at 4°C against 20 mM acetate buffer (pH 5.0) containing 0.05% NONIDET P-40 (to be referred to as NP-40 hereinafter). The precipitate thus formed was removed by centrifugation, and the resulting supernatant was subjected to a chromatofocasing column chromatography. That is, a sample was applied to a column (2 cm in diameter x 70 cm in length) which has been packed with a chromatofocasing gel (Pharmacia) and equilibrated with 10 mM Tris-HCl (pH 9.0)/0.05% NP-40, the resulting column was washed with 20 mM acetate buffer (pH 4.75)/0.05% NP-40/10% Polybuffer 74 (Pharmacia), and then elution was carried out by linear gradient of 20 to 250 mM acetate buffer (pH 4.3) prepared using 10% Polybuffer 74. The positive fractions by immunoassay of MCP shown in Reference Example 4 were pooled and dialyzed against 10 mM phosphate-buffered saline (to be referred to as PBS hereinafter, pH 7.4)/0.05% NP-40. The thus dialyzed sample was subjected to an affinity column chromatography using an anti-MCP monoclonal antibody as a ligand whose preparation method was shown in Reference Example 2. The sample was applied to an affinity column (2 cm in diameter x 10 cm in length) and washed with PBS (pH 7.4)/0.05% NP-40 and then with PBS (pH 7.4)/0.5 M NaCl/0.05% NP-40. The protein of interest was eluted with 0.17 M glycine-HCl buffer (pH 2.5)/0.05% NP-40, and 10 ml of the elution fraction was immediately neutralized with 1 ml of 1 M Tris. The thus neutralized fraction was dialyzed overnight against 20 mM phosphate buffer (pH 6.0)/0.05% NP-40 and then concentrated using a concentration apparatus (Ms.BTAURY-KN, Atto Corp.). By the process of Example 6, the protein of the present invention was purified to such a level that silver staining after its SDS-PAGE showed a single band, with a recovery yield of 70% or more.

### (Example 7) Reaction with C3b on a solid phase

It has been revealed by Seya *et al.* that natural type MCP extracted from cell membranes shows a cofactor activity of factor I on C3b in a liquid phase but not on C3b on a solid phase (Seya, T. et al., *Biochem. J.,* 264, 581 - 588, 1989). In consequence, the inventors of the present invention have examined the protein obtained in Example 6 about its action upon C3b on a solid phase. The procedure disclosed in the Seya *et al.* report was used in this examination.

Firstly, each complement component was purified in accordance with the following known methods.
C1: Matsumoto, M. et al., *J. Immunol.,* 142, 2743 - 2750, 1989
C2: Nagasawa, S. et al., *Proc. Natl. Acad. Sci. USA,* 74, 2998 - 3001, 1977
C3: Seya, T. et al., *J. Biochem.,* 89, 659 - 664, 1981
C4: Nagasawa, S. et al., *Molec. Immunol.,* 17, 1365 - 1372, 1980
B : Seya, T. et al., *Complement,* 2, 165 - 174, 1985
D : Volanakis, J.E. et al., *J. Immunol.,* 119, 337 - 342, 1977
I : Nagasawa, S. et al., *J. Immunol.,* 125, 578 - 582, 1980
A portion of C3 thus purified was labeled with ¹²⁵I using a periodic acid labeling reagent (Pierce Chemical Co.).

In order to obtain C3b adhered on erythrocyte membrane, rinsed sheep erythrocyte (E, Nikken Seibutsu) and anti-sheep erythrocyte rabbit antibody (A, Cordis) were allowed to react each other, followed by the addition of C1, C4 and C2 in that order to obtain EAC4b2 complex. To this was added a small amount of unlabeled C3 to form of EAC4b2a3b, followed by the addition of ¹²⁵I-labeled C3, factor B and factor D to obtain EA¹²⁵IC3b (¹²⁵I-labeled C3b adhered on erythrocyte membrane). The resulting erythrocytes with a cell count of 1 x 10⁷ - 3 x 10⁷ were used as the substrate, and 1 µg of factor I and 40 ng of the protein obtained in Example 6 were added to the substrate and, after adjusting the total volume to 160 µl, the mixture was allowed to stand still for 1 hour at 37°C. The reaction was terminated by adding 10 µl of each of 3% NP-40 and 10% sodium dodecyl sulfate (SDS), 5 µl of β-mercaptoethanol and 45 µl of a chromogen mixture for SDS-PAGE use, and the thus treated reaction mixture was subjected to SDS-PAGE and then to autoradiography to analyze decomposed products of C3b on the solid phase. In this instance, a natural type MCP was purified from a cell membrane fraction of HSB-2 cell line in accordance with the procedure described in Example 6 to be used as a control of the cofactor ability of C3b decomposition, and, since it does not react with C3b on a solid phase, its reaction was carried out after dissolving erythrocyte membranes in advance by adding NP-40 to a concentration of 0.4% at the starting time of the reaction, thereby allowing C3b to exist in a liquid phase.

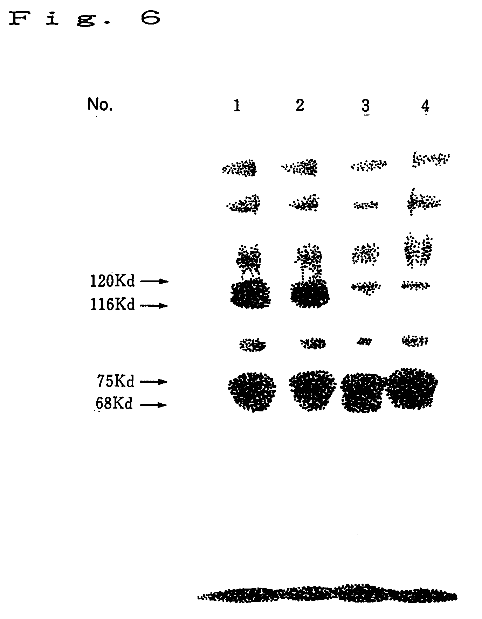

The results are shown in Fig. 6. Fig. 6 is an SDS-PAGE autoradiogram of samples obtained after various treatments of the ¹²⁵I-labeled C3b. Treatment conditions of samples on respective lanes are shown below.
- Lane 1:: solid phase C3b + factor I
- Lane 2:: solid phase C3b + factor I + natural type MCP
- Lane 3:: solid phase C3b + factor I + natural type MCP + NP-40 (NP-40 was added to solubilize C3b on the solid phase)
- Lane 4:: solid phase C3b + factor I + inventive protein
Comparison of lanes 1 and 2 reveals no changes in the C3b band pattern. This indicates that the natural type MCP does not have an activity as a cofactor of factor I upon C3b on the solid phase. In lane 3 in which NP-40 was further added, disappearance of a band of 116 kd to 120 kd can be observed when compared with lane 2. This indicates that the C3b solubilized by NP-40 is decomposed by factor I which uses natural type MCP as its cofactor.

In lane 4 in which C3b on the solid phase was allowed to react with factor I and the protein of the present invention in the absence of NP-40, disappearance of a band of 116 kd to 120 kd can be observed similar to the case of lane 3. This indicates that the protein of the present invention, unlike the case of the natural type MCP, can act as a cofactor of factor I even upon the solid phase C3b. Thus, it has been revealed that the protein of the present invention can exhibit a cofactor activity even upon C3b on a solid phase in the same manner as the case of the activity of natural type MCP upon C3b in a liquid phase.
(Example 8) Effects on complement-dependent cytotoxicity
prevention of cytotoxic effects by the protein of the present invention was evaluated using CHO cells which show sensitivity to cytotoxic action by the human complement system. That is, CHO-K1 cells (ATCC CCL61) were cultured in the usual way using Ham's F12 medium (Nissui Pharmaceutical Co., Ltd.) containing 10% bovine serum (Cell Culture Laboratories). The anti-CHO cell rabbit antiserum shown in Reference Example 5 was added to a sample of about 1 x 10⁶ cells, and the mixture was treated at 0°C for 15 minutes. To this were added normal human serum prepared to a concentration of 20% using a gelatin veronal buffer (0.145 M NaCl/0.05 M sodium barbital (pH 7.4)/0.1% gelatin/100 mM EGTA) containing 100 mM EGTA and 10 mM MgCl₂ and about 400 ng of the inventive protein having a novel pharmacological property, and the mixture was allowed to stand still for 1 hour at 37°C. The thus treated sample was mixed with anti-human iC3b mouse monoclonal antibody, treated at 0°C for 45 minutes, mixed further with FITC-labeled anti-mouse IgG goat antibody (Cappel) and propidium iodide and treated again at 0°C for 30 minutes. In this instance, the anti-human iC3b mouse monoclonal antibody (*Immunology,* 62, 413 - 417, 1987) was obtained from Dr. K. Iida at New York University. The number of viable and dead cells and fluorescence intensity (proportional to the C3b deposition on CHO cells) in the thus treated samples were analyzed using a fluorescence activation cell sorter (EPICS ProfileII, Coaltar).

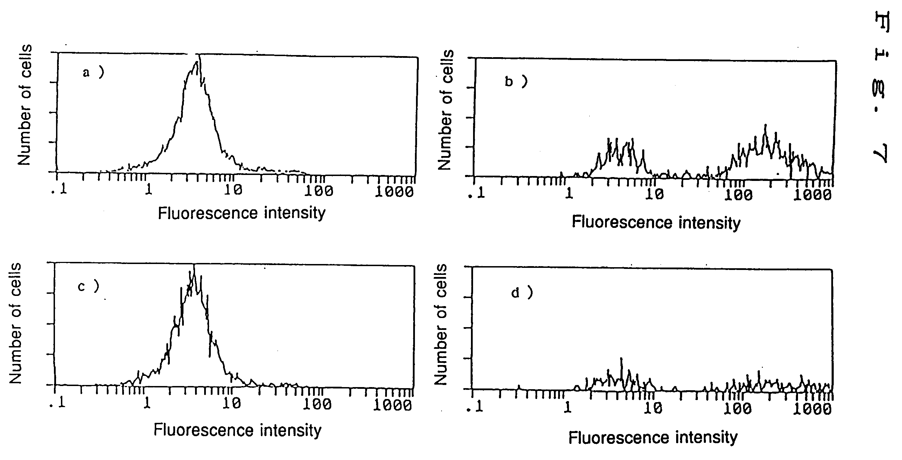

The results are shown in Fig. 7. Experimental methods are as described in Example 8. In the figure, a) shows viable cells and b) shows dead cells, both in the control group (in the absence of MCP). Also, c) shows viable cells and d) shows dead cells, both in the presence of the protein of the present invention. In each graph, an index showing the number of cells is plotted as ordinate, and the fluorescence intensity as abscissa. It is evident from this figure that a large number of cells with iC3b deposition (iC3b fixed cells) (peaks on the high fluorescence intensity side) are detected in the dead cell fraction [Fig. 7 (b)] in the absence of the protein of the present invention, while iC3b fixed cells are reduced sharply and dead cells themselves also decrease in the presence of the MCP molecules of the present invention [Fig. 7 (d)].

When these results are taken into consideration together with the results of Example 7, it seems that, unlike the case of natural type MCP, the protein of the present invention reduces absolute amount of C3b deposition on the cells by C3b decomposition even on the solid phase and, as the results, inhibits cytotoxic nature of the complement system.

### (Example 9) Toxicity test

Toxicity tests in mice and rats were carried out using the protein of the present invention obtained in Example 6. That is, the protein obtained in Example 6 was intravenously administered once a day for one week to 6- to 8-week-old 10 female and 10 male mice of Balb/c line and to 10 female and 10 male rats of Wistar line of the same age. General conditions of each animal were observed through the administration period. In these tests, no mortal case was found and no changes in general conditions were observed in each animal even in the group of the highest dose of 10 mg/kg.

### (Example 10) Preparation of solutions for injection use

A 100 mg portion of the protein of the present invention obtained in Example 6 was dissolved in 100 ml of physiological saline containing 10 mg/ml of hydrolyzed gelatin, and the solution was sterilized by filtration using a filter of 0.22 µm in pore size (Mylex GV, Millipore Corp.). The thus prepared solution was aseptically dispensed in 10 ml portions into glass vials and sealed to be used as solutions for injection use.

### (Example 11) Preparation of freeze-dried powders for injection use

A 100 mg portion of the protein of the present invention obtained in Example 6 was dissolved in 100 ml of 10 mM PBS (pH 7.4) containing 100 mg/ml of human serum albumin, and the solution was sterilized by filtration using a filter of 0.22 µm in pore size (Mylex GV, Millipore Corp.). The thus prepared solution was aseptically dispensed in 3 ml portions into glass vials, freeze-dried and then sealed to be used as freeze-dried powders for injection use.

### (Reference Example 1) Preparation of anti-MCP monoclonal antibody

An anti-MCP monoclonal antibody was prepared basically in accordance with the procedure of Kohler *et al.* (Kohler, G. et al., *Nature,* 256, 495 - 497, 1975). Illustrative methods have been reported by Seya, T. et al., in *Complement Inflamm.,* 7, 78 - 89, 1990. That is, a natural type MCP purified from a cell membrane fraction (0.5 µg/0.5 ml PBS/0.002% NP-40) was mixed thoroughly with 0.8 ml of Freund's complete adjuvant and subcutaneously administered to Balb/c mice. The administration was carried out three times at an interval of 10 days. Spleens were excised from the mice 4 days after the final administration to isolate splenocytes which were subsequently fused with mouse myeroma NS-1 cells in the usual way. Culture filtrates obtained from clones of these fused cells were screened by a protein A rosette formation method using sheep erythrocytes, thereby obtaining anti-MCP antibody producing clones. These hybridoma cells were transplanted in the peritoneal cavity of Balb/c mice which were subsequently exposed to radial rays of 400 rad. Ten days thereafter, peritoneal fluid was collected from the mice. Ammonium sulfate was added to the peritoneal fluid to a 50% saturation to carry out salting out. Four hours thereafter, the precipitate was recovered by centrifugation, dissolved in water and then dialyzed against 0.5 M NaCl/0.02 M Tris-HCl (pH 8.9) to be used as a crude antibody purification fraction. The crude antibody purification fraction was applied to a column packed with protein A Sepharose (Pharmacia) which has been equilibrated in advance with 2 M NaCl/0.1 M glycine/0.02 M Tris-HCl (pH 8.9), the column was washed with the equilibration buffer and then the antibodies were eluted in succession using 0.1 M citric acid (pH 6.0) and 0.1 M citric acid (pH 3.0). Elution of antibodies was monitored by the ultraviolet ray absorbance at 280 nm (UV₂₈₀). Thereafter, elution peaks were collected and dialyzed against PBS to be used as a purified product of anti-MCP monoclonal antibody.

### (Reference Example 2) Preparation of an affinity carrier using monoclonal antibody as ligand

A 100 mg portion of the purified product of anti-MCP monoclonal antibody obtained in Reference Example 1 was mixed with 50 ml of BrCN activated Sepharose 4B (Pharmacia), and the mixture was gently stirred overnight at 4°C. Thereafter, the mixture was washed with 1 M Tris-HCl (pH 8.5) and then equilibrated with a 0.5 M NaCl/20 mM phosphate buffer solution (pH 7.5).

### (Reference Example 3) Preparation of enzyme-labeled monoclonal antibody

In order to construct an MCP enzyme immunoassay system, an enzyme-labeled antibody was prepared using the monoclonal antibody obtained in Reference Example 1. Firstly, 0.2 ml of 0.1 M NaIO₄ was added to 1 ml of 4 mg/ml solution of horseradish peroxidase (Toyobo) and mixed, and the mixture was then allowed to stand still for 15 minutes at 25°C. To this was added 0.2 ml of 1.5% ethylene glycol aqueous solution, followed by mixing and additional standing for 20 minutes at 25°C. Thereafter, the mixture was dialyzed three times against 1 mM sodium acetate buffer solution (pH 4.4). To this were added 70 µl of 0.2 M sodium carbonate buffer (20 x SCB, pH 9.5) and, immediately thereafter, a solution of the antibody (prepared by dialyzing three times against 2 x SCB and then dissolving to a concentration of about 5 mg/ml), followed by mixing and subsequent standing at 25°C for 2 hours. Next, to this was added 0.1 ml of NaBH₄ solution adjusted to a concentration of 4 mg/ml with 1 x SCB, and the mixture was allowed to stand still for 2 hours at 4°C. Thereafter, the thus treated sample was dialyzed three times against 76 mM PBS (pH 6.4) to be used as an enzyme-labeled antibody sample.

### (Reference Example 4) Enzyme immunoassay of MCP

As a first antibody, 5 µg/ml solution of one of the monoclonal antibodies obtained in Reference Example 1 was dispensed in 0.1 ml portions into wells of a 96 well multititer immunoplate (Nunc), and the plate was allowed to stand still overnight at 4°C. After washing each well 5 times with 0.3 ml of ion-exchanged water, 0.1 ml of a sample or a standard substance mixed with the same amount of PBS containing 10% bovine serum albumin was added to each well and then maintained at room temperature for 2 hours. Each well was washed 5 times with 0.3 ml of 0.005% Tween 20-containing physiological saline (washing solution) and then once with 0.3 ml of ion-exchanged water. A 0.1 ml portion of a mixture consisting of the horseradish peroxidase-labeled monoclonal antibody (0.5 µg/ml) obtained in Reference Example 3 as a second antibody and the same volume of PBS containing 10% bovine serum albumin was added to each well and then maintained at room temperature for 2 hours. After washing each well 5 times with 0.3 ml of the washing solution and then once with 0.3 ml of ion-exchanged water, 0.1 ml of a color developing solution (prepared by dissolving 45 mg of orthophenylenediamine and 400 µl of 1% H₂O₂ in 15 ml of sodium phosphate-citric acid buffer, pH 5.0) was added to each well and maintained at room temperature for 15 minutes. Thereafter, 0.1 ml of a reaction termination solution (1 N H₂SO₄) was added to each well and mixed, and absorbance of the reaction solution was measured at 490 nm (OD₄₉₀) using a microplate reader (ETY-96A, Oriental Instruments, Ltd.). The amount of MCP in each sample was calculated using a calibration curve obtained from OD₄₉₀ values of standard substances.

### (Reference Example 5) Preparation of anti-CHO cell rabbit antiserum

CHO-K1 cells cultured in the usual way were washed thoroughly with PBS and then made into a cell suspension with a cell density of about 1 x 10⁷/ml. About 2 ml of the cell suspension was intraarterially administered to Japanese white rabbits. The administration was carried out five times at an interval of 1 week, and blood samples were collected from ear arteries of the rabbits one week after the final administration. After isolating sera, salting-out was carried out in the same manner as described in Reference Example 1. The resulting precipitate was recovered, dissolved in an appropriate volume of water and then dialyzed against PBS. This was used as an anti-CHO cell rabbit antiserum preparation.

### INDUSTRIAL APPLICATION FIELD

The present invention provides a protein having a novel pharmacological property which has a function as a cofactor of factor I even against C3b on a solid phase similar to the case of C3b in a liquid phase.

This is a novel protein which can inhibit actual state of injurious actions of the complement system, namely actions of complement system components adsorbed to a solid phase substrate (cell membrane).

This invention also provides a DNA fragment which encodes the inventive protein and a pharmaceutical composition which contains the inventive protein as an active ingredient.

In consequence, the present invention opens a way for the treatment of diseases in which the complement system exerts injurious actions on the host cells, such as nephritis, hepatitis, arteritis, asthma, autoimmune disease, ischemic organ disorder, reperfusion injury, rheumatoid arthritis, graft rejection, shock, multiorgan failure, disseminated intravascular coagulation syndrome and adult respiratory distress syndrome (ARDS), as well as ARDS-like disease and shock caused by hemodialysis and burn shock.

[SEQUENCE LISTING]
Sequence ID No.: 1
Sequence Length: 279
Sequence Type: amino acid
Topology: linear
Molecular Type: protein

Sequence
Cys Glu Glu Pro Pro Thr Phe Glu Ala Met Glu Leu Ile Gly Lys Pro
1               5                   10                  15
Lys Pro Tyr Tyr Glu Ile Gly Glu Arg Val Asp Tyr Lys Cys Lys Lys
            20                  25                  30
Gly Tyr Phe Tyr Ile Pro Pro Leu Ala Thr His Thr Ile Cys Asp Arg
        35                  40                  45
Asn His Thr Trp Leu Pro Val Ser Asp Asp Ala Cys Tyr Arg Glu Thr
    50                  55                  60
Cys Pro Tyr Ile Arg Asp Pro Leu Asn Gly Gln Ala Val Pro Ala Asn
65                  70                  75                  80
Gly Thr Tyr Glu Phe Gly Tyr Gln Met His Phe Ile Cys Asn Glu Gly
                85                  90                  95
Tyr Tyr Leu Ile Gly Glu Glu Ile Leu Tyr Cys Glu Leu Lys Gly Ser
            100                 105                 110
Val Ala Ile Trp Ser Gly Lys Pro Pro Ile Cys Glu Lys Val Leu Cys
        115                 120                 125
Thr Pro Pro Pro Lys Ile Lys Asn Gly Lys His Thr Phe Ser Glu Val
    130                 135                 140
Glu Val Phe Glu Tyr Leu Asp Ala Val Thr Tyr Ser Cys Asp Pro Ala
145                 150                 155                 160

Pro Gly Pro Asp Pro Phe Ser Leu Ile Gly Glu Ser Thr Ile Tyr Cys
                165                 170                 175

Gly Asp Asn Ser Val Trp Ser Arg Ala Ala Pro Glu Cys Lys Val Val
            180                 185                 190

Lys Cys Arg Phe Pro Val Val Glu Asn Gly Lys Gln Ile Ser Gly Phe
        195                 200                 205

Gly Lys Lys Phe Tyr Tyr Lys Ala Thr Val Met Phe Glu Cys Asp Lys
    210                 215                 220

Gly Phe Tyr Leu Asp Gly Ser Asp Thr Ile Val Cys Asp Ser Asn Ser
225                 230                 235                 240

Thr Trp Asp Pro Pro Val Pro Lys Cys Leu Lys Gly Pro Arg Pro Thr
                245                 250                 255

Tyr Lys Pro Pro Val Ser Asn Tyr Pro Gly Tyr Pro Lys Pro Glu Glu
            260                 265                 270

Gly Ile Leu Asp Ser Leu Asp
        275             279

Sequence ID No.: 2

Sequence Length: 251

Sequence Type: amino acid

Topology: linear

Molecular Type: protein

Sequence

Cys Glu Glu Pro Pro Thr Phe Glu Ala Met Glu Leu Ile Gly Lys Pro
1               5                   10                  15

Lys Pro Tyr Tyr Glu Ile Gly Glu Arg Val Asp Tyr Lys Cys Lys Lys
            20                  25                  30

Gly Tyr Phe Tyr Ile Pro Pro Leu Ala Thr His Thr Ile Cys Asp Arg
        35                  40                  45

Asn His Thr Trp Leu Pro Val Ser Asp Asp Ala Cys Tyr Arg Glu Thr
    50                  55                  60

Cys Pro Tyr Ile Arg Asp Pro Leu Asn Gly Gln Ala Val Pro Ala Asn
65                  70                  75                  80

Gly Thr Tyr Glu Phe Gly Tyr Gln Met His Phe Ile Cys Asn Glu Gly
                85                  90                  95

Tyr Tyr Leu Ile Gly Glu Glu Ile Leu Tyr Cys Glu Leu Lys Gly Ser
            100                 105                 110

Val Ala Ile Trp Ser Gly Lys Pro Pro Ile Cys Glu Lys Val Leu Cys
        115                 120                 125

Thr Pro Pro Pro Lys Ile Lys Asn Gly Lys His Thr Phe Ser Glu Val
    130                 135                 140

Glu Val Phe Glu Tyr Leu Asp Ala Val Thr Tyr Ser Cys Asp Pro Ala
145                 150                 155                 160

Pro Gly Pro Asp Pro Phe Ser Leu Ile Gly Glu Ser Thr Ile Tyr Cys
                165                 170                 175
Gly Asp Asn Ser Val Trp Ser Arg Ala Ala Pro Glu Cys Lys Val Val
            180                 185                 190
Lys Cys Arg Phe Pro Val Val Glu Asn Gly Lys Gln Ile Ser Gly Phe
        195                 200                 205
Gly Lys Lys Phe Tyr Tyr Lys Ala Thr Val Met Phe Glu Cys Asp Lys
    210                 215                 220
Gly Phe Tyr Leu Asp Gly Ser Asp Thr Ile Val Cys Asp Ser Asn Ser
225                 230                 235                 240
Thr Trp Asp Pro Pro Val Pro Lys Cys Leu Lys
                245                 250 251

Sequence ID No.: 3
Sequence Length: 840
Sequence Type: nucleic acid
Strandedness: double
Topology: linear
Molecular Type: cDNA to mRNA
Sequence characteristics
    Designation: mat peptide
    Location: 1 ·· 840
    Method determined: S

Sequence

TGTGAGGAGC CACCAACATT TGAAGCTATG GAGCTCATTG GTAAACCAAA ACCCTACTAT 60

GAGATTGGTG AACGAGTAGA TTATAAGTGT AAAAAAGGAT ACTTCTATAT ACCTCCTCTT 120

GCCACCCATA CTATTTGTGA TCGGAATCAT ACATGGCTAC CTGTCTCAGA TGACGCCTGT 180

TATAGAGAAA CATGTCCATA TATACGGGAT CCTTTAAATG GCCAAGCAGT CCCTGCAAAT 240

GGGACTTACG AGTTTGGTTA TCAGATGCAC TTTATTTGTA ATGAGGGTTA TTACTTAATT 300

GGTGAAGAAA TTCTATATTG TGAACTTAAA GGATCAGTAG CAATTTGGAG CGGTAAGCCC 360

CCAATATGTG AAAAGGTTTT GTGTACACCA CCTCCAAAAA TAAAAAATGG AAAACACACC 420

TTTAGTGAAG TAGAAGTATT TGAGTATCTT GATGCAGTAA CTTATAGTTG TGATCCTGCA 480

CCTGGACCAG ATCCATTTTC ACTTATTGGA GAGAGCACGA TTTATTGTGG TGACAATTCA 540

GTGTGGAGTC GTGCTGCTCC AGAGTGTAAA GTGGTCAAAT GTCGATTTCC AGTAGTCGAA 600

AATGGAAAAC AGATATCAGG ATTTGGAAAA AAATTTTACT ACAAAGCAAC AGTTATGTTT 660

GAATGCGATA AGGGTTTTTA CCTCGATGGC AGCGACACAA TTGTCTGTGA CAGTAACAGT 720

ACTTGGGATC CCCCAGTTCC AAAGTGTCTT AAAGGTCCTA GGCCTACTTA CAAGCCTCCA 780

GTCTCAAATT ATCCAGGATA TCCTAAACCT GAGGAAGGAA TACTTGACAG TTTGGATTGA 840

Sequence ID No.: 4

Sequence Length: 756

Sequence Type: nucleic acid

Strandedness: double

Topology: linear

Molecular Type: cDNA to mRNA

Sequence characteristics

Designation: mat peptide

Location: 1 ·· 756

Method determined: S

Sequence

TGTGAGGAGC CACCAACATT TGAAGCTATG GAGCTCATTG GTAAACCAAA ACCCTACTAT 60

GAGATTGGTG AACGAGTAGA TTATAAGTGT AAAAAAGGAT ACTTCTATAT ACCTCCTCTT 120

GCCACCCATA CTATTTGTGA TCGGAATCAT ACATGGCTAC CTGTCTCAGA TGACGCCTGT 180

TATAGAGAAA CATGTCCATA TATACGGGAT CCTTTAAATG GCCAAGCAGT CCCTGCAAAT 240

GGGACTTACG AGTTTGGTTA TCAGATGCAC TTTATTTGTA ATGAGGGTTA TTACTTAATT 300

GGTGAAGAAA TTCTATATTG TGAACTTAAA GGATCAGTAG CAATTTGGAG CGGTAAGCCC 360

CCAATATGTG AAAAGGTTTT GTGTACACCA CCTCCAAAAA TAAAAAATGG AAAACACACC 420

TTTAGTGAAG TAGAAGTATT TGAGTATCTT GATGCAGTAA CTTATAGTTG TGATCCTGCA 480

CCTGGACCAG ATCCATTTTC ACTTATTGGA GAGAGCACGA TTTATTGTGG TGACAATTCA 540

GTGTGGAGTC GTGCTGCTCC AGAGTGTAAA GTGGTCAAAT GTCGATTTCC AGTAGTCGAA 600

AATGGAAAAC AGATATCAGG ATTTGGAAAA AAATTTTACT ACAAAGCAAC AGTTATGTTT 660

GAATGCGATA AGGGTTTTTA CCTCGATGGC AGCGACACAA TTGTCTGTGA CAGTAACAGT 720

ACTTGGGATC CCCCAGTTCC AAAGTGTCTT AAATGA 756

## Claims

1. A protein having a novel pharmacological property which comprises a natural type membrane cofactor protein (MCP) from which at least its transmembrane region and cytoplasmic tail region are deleted.

2. The protein having a novel pharmacological property according to claim 1 wherein said protein contains at least a portion of an amino acid sequence represented by the following formula 1.

(formula 1)

Cys Glu Glu Pro Pro Thr Phe Glu Ala Met Glu Leu Ile Gly Lys Pro
1               5                   10                  15

Lys Pro Tyr Tyr Glu Ile Gly Glu Arg Val Asp Tyr Lys Cys Lys Lys
            20                  25                  30

Gly Tyr Phe Tyr Ile Pro Pro Leu Ala Thr His Thr Ile Cys Asp Arg
        35                  40                  45

Asn His Thr Trp Leu Pro Val Ser Asp Asp Ala Cys Tyr Arg Glu Thr
    50                  55                  60

Cys Pro Tyr Ile Arg Asp Pro Leu Asn Gly Gln Ala Val Pro Ala Asn
65                  70                  75                  80

Gly Thr Tyr Glu Phe Gly Tyr Gln Met His Phe Ile Cys Asn Glu Gly
                85                  90                  95

Tyr Tyr Leu Ile Gly Glu Glu Ile Leu Tyr Cys Glu Leu Lys Gly Ser
            100                 105                 110

Val Ala Ile Trp Ser Gly Lys Pro Pro Ile Cys Glu Lys Val Leu Cys
        115                 120                 125

Thr Pro Pro Pro Lys Ile Lys Asn Gly Lys His Thr Phe Ser Glu Val
    130                 135                 140

Glu Val Phe Glu Tyr Leu Asp Ala Val Thr Tyr Ser Cys Asp Pro Ala

145                 150                 155                 160

Pro Gly Pro Asp Pro Phe Ser Leu Ile Gly Glu Ser Thr Ile Tyr Cys
                165                 170                 175

Gly Asp Asn Ser Val Trp Ser Arg Ala Ala Pro Glu Cys Lys Val Val
            180                 185                 190

Lys Cys Arg Phe Pro Val Val Glu Asn Gly Lys Gln Ile Ser Gly Phe
        195                 200                 205

Gly Lys Lys Phe Tyr Tyr Lys Ala Thr Val Met Phe Glu Cys Asp Lys
    210                 215                 220

Gly Phe Tyr Leu Asp Gly Ser Asp Thr Ile Val Cys Asp Ser Asn Ser
225                 230                 235                 240

Thr Trp Asp Pro Pro Val Pro Lys Cys Leu Lys Gly Pro Arg Pro Thr
                245                 250                 255

Tyr Lys Pro Pro Val Ser Asn Tyr Pro Gly Tyr Pro Lys Pro Glu Glu
            260                 265                 270

Gly Ile Leu Asp Ser Leu Asp
        275                 279

3. The protein having a novel pharmacological property according to claim 1 wherein said protein contains an amino acid sequence represented by the aforementioned formula 1 or the following formula 2.

(formula 2)
Cys Glu Glu Pro Pro Thr Phe Glu Ala Met Glu Leu Ile Gly Lys Pro
1               5                   10                  15
Lys Pro Tyr Tyr Glu Ile Gly Glu Arg Val Asp Tyr Lys Cys Lys Lys
            20                  25                  30
Gly Tyr Phe Tyr Ile Pro Pro Leu Ala Thr His Thr Ile Cys Asp Arg
        35                  40                  45
Asn His Thr Trp Leu Pro Val Ser Asp Asp Ala Cys Tyr Arg Glu Thr
    50                  55                  60

Cys Pro Tyr Ile Arg Asp Pro Leu Asn Gly Gln Ala Val Pro Ala Asn
65                  70                  75                  80

Gly Thr Tyr Glu Phe Gly Tyr Gln Met His Phe Ile Cys Asn Glu Gly
                85                  90                  95

Tyr Tyr Leu Ile Gly Glu Glu Ile Leu Tyr Cys Glu Leu Lys Gly Ser
            100                 105                 110

Val Ala Ile Trp Ser Gly Lys Pro Pro Ile Cys Glu Lys Val Leu Cys
        115                 120                 125

Thr Pro Pro Pro Lys Ile Lys Asn Gly Lys His Thr Phe Ser Glu Val
    130                 135                 140

Glu Val Phe Glu Tyr Leu Asp Ala Val Thr Tyr Ser Cys Asp Pro Ala
145                 150                 155                 160

Pro Gly Pro Asp Pro Phe Ser Leu Ile Gly Glu Ser Thr Ile Tyr Cys
                165                 170                 175

Gly Asp Asn Ser Val Trp Ser Arg Ala Ala Pro Glu Cys Lys Val Val
            180                 185                 190

Lys Cys Arg Phe Pro Val Val Glu Asn Gly Lys Gln Ile Ser Gly Phe
        195                 200                 205

Gly Lys Lys Phe Tyr Tyr Lys Ala Thr Val Met Phe Glu Cys Asp Lys
    210                 215                 220

Gly Phe Tyr Leu Asp Gly Ser Asp Thr Ile Val Cys Asp Ser Asn Ser
225                 230                 235                 240

Thr Trp Asp Pro Pro Val Pro Lys Cys Leu Lys
                245                 250 251

4. The protein having a novel pharmacological property according to claim 1 wherein said protein comprises of an amino acid sequence represented by the aforementioned formula 1 or 2.

5. The protein having a novel pharmacological property according to any one of claims 1 to 4 wherein said protein has a function of a cofactor of factor I against C3b on a solid phase.

6. The protein having a novel pharmacological property according to any one of claims 1 to 5 wherein said protein is produced by cells excluding human cells.

7. The protein having a novel pharmacological property according to claim 6 wherein said cells excluding human cells are *Escherichia coli* cells.

8. The protein having a novel pharmacological property according to claim 6 wherein said cultured cells are mammalian cultured cells.

9. The protein having a novel pharmacological property according to any one of claims 6 to 8 wherein said protein is produced making use of at least a portion of a DNA fragment having a nucleotide sequence represented by the following formula 3.

(formula 3)

TGTGAGGAGC CACCAACATT TGAAGCTATG GAGCTCATTG GTAAACCAAA ACCCTACTAT 60

GAGATTGGTG AACGAGTAGA TTATAAGTGT AAAAAAGGAT ACTTCTATAT ACCTCCTCTT 120

GCCACCCATA CTATTTGTGA TCGGAATCAT ACATGGCTAC CTGTCTCAGA TGACGCCTGT 180

TATAGAGAAA CATGTCCATA TATACGGGAT CCTTTAAATG GCCAAGCAGT CCCTGCAAAT 240

GGGACTTACG AGTTTGGTTA TCAGATGCAC TTTATTTGTA ATGAGGGTTA TTACTTAATT 300

GGTGAAGAAA TTCTATATTG TGAACTTAAA GGATCAGTAG CAATTTGGAG CGGTAAGCCC 360

CCAATATGTG AAAAGGTTTT GTGTACACCA CCTCCAAAAA TAAAAAATGG AAAACACACC 420

TTTAGTGAAG TAGAAGTATT TGAGTATCTT GATGCAGTAA CTTATAGTTG TGATCCTGCA 480

CCTGGACCAG ATCCATTTTC ACTTATTGGA GAGAGCACGA TTTATTGTGG TGACAATTCA 540

GTGTGGAGTC GTGCTGCTCC AGAGTGTAAA GTGGTCAAAT GTCGATTTCC AGTAGTCGAA 600
AATGGAAAAC AGATATCAGG ATTTGGAAAA AAATTTTACT ACAAAGCAAC AGTTATGTTT 660
GAATGCGATA AGGGTTTTTA CCTCGATGGC AGCGACACAA TTGTCTGTGA CAGTAACAGT 720
ACTTGGGATC CCCCAGTTCC AAAGTGTCTT AAAGGTCCTA GGCCTACTTA CAAGCCTCCA 780
GTCTCAAATT ATCCAGGATA TCCTAAACCT GAGGAAGGAA TACTTGACAG TTTGGATTGA 840

10. A DNA fragment having a nucleotide sequence which encodes the protein having a novel pharmacological property of any one of the claims 1 to 8.

11. The DNA fragment according to claim 10 wherein said fragment has at least a portion of a nucleotide sequence represented by the aforementioned formula 3.

12. A DNA fragment which has a nucleotide sequence represented by the aforementioned formula 3.

13. A DNA fragment which has a nucleotide sequence represented by the following formula 4.

(formula 4)

TGTGAGGAGC CACCAACATT TGAAGCTATG GAGCTCATTG GTAAACCAAA ACCCTACTAT 60

GAGATTGGTG AACGAGTAGA TTATAAGTGT AAAAAAGGAT ACTTCTATAT ACCTCCTCTT 120

GCCACCCATA CTATTTGTGA TCGGAATCAT ACATGGCTAC CTGTCTCAGA TGACGCCTGT 180

TATAGAGAAA CATGTCCATA TATACGGGAT CCTTTAAATG GCCAAGCAGT CCCTGCAAAT 240

GGGACTTACG AGTTTGGTTA TCAGATGCAC TTTATTTGTA ATGAGGGTTA TTACTTAATT 300

GGTGAAGAAA TTCTATATTG TGAACTTAAA GGATCAGTAG CAATTTGGAG CGGTAAGCCC 360

CCAATATGTG AAAAGGTTTT GTGTACACCA CCTCCAAAAA TAAAAAATGG AAAACACACC 420

TTTAGTGAAG TAGAAGTATT TGAGTATCTT GATGCAGTAA CTTATAGTTG TGATCCTGCA 480

CCTGGACCAG ATCCATTTTC ACTTATTGGA GAGAGCACGA TTTATTGTGG TGACAATTCA 540

GTGTGGAGTC GTGCTGCTCC AGAGTGTAAA GTGGTCAAAT GTCGATTTCC AGTAGTCGAA 600

AATGGAAAAC AGATATCAGG ATTTGGAAAA AAATTTTACT ACAAAGCAAC AGTTATGTTT 660

GAATGCGATA AGGGTTTTTA CCTCGATGGC AGCGACACAA TTGTCTGTGA CAGTAACAGT 720

ACTTGGGATC CCCCAGTTCC AAAGTGTCTT AAATGA 756

14. A pharmaceutical composition for use in the treatment of diseases in which the complement system exerts injurious reaction upon the host cells, which contains the protein having a novel pharmacologiocal property of any one of the claims 1 to 9 as at least one of active ingredients.

15. A process which comprises using the protein having a novel pharmacologiocal property of any one of the claims 1 to 9 for the production of therapeutic preparations of diseases in which the complement system exerts injurious reaction upon the host cells.

16. The pharmaceutical composition according to claim 14 wherein said disease is at least one disease selected from the group consisting of nephritis, hepatitis, arteritis, asthma, autoimmune disease, ischemic organ disorder, reperfusion injury, rheumatoid arthritis, graft rejection, shock, multiorgan failure, disseminated intravascular coagulation syndrome, adult respiratory distress syndrome (ARDS) and ARDS-like disease shock caused by hemodialysis or burn shock.
